# FASCICULE DE BREVET EUROPEEN

(11) **EP 2 198 927 B1**
(45) Date de publication et mention de la délivrance du brevet: **06.03.2019**
(21) Numéro de dépôt: 09179996.5
(22) Date de dépôt: 18.12.2009
(51) Int. Cl.: A61Q 5/06, A61Q 5/08, A61Q 5/10, A61K 8/22, A61K 8/31, A61K 8/34, A61K 8/37, A61K 8/39, A61K 8/86, A61K 8/92

(54) **Composition comprenant un corps gras et un tensioactif oxyethylène particulier et procédé de coloration la mettant en oeuvre**
Zusammensetzung enthaltend einen Fettstoff und ein spezielles ethoxyliertes Tensid und Verfahren zum Färben diese gebrauchend
Composition comprising a fatty substance and a particular oxyethylene surfactant and dyeing process using it

(30) Priorité: 19.12.2008 FR 0858881
(43) Date de publication de la demande: 23.06.2010
(73) Titulaire: L'Oréal, 75008 Paris (FR)
(72) Inventeur: Hercouet, Leïla, 93360, Neuilly Plaisance (FR); Simonet, Frédéric, 92110, Clichy (FR); Audousset, Marie-Pascale, 92600, Asnieres (FR); Schlosser, Isabelle, 75009, Paris (FR)
(74) Mandataire: Wattremez, Catherine

(56) Documents cités:
- EP-A1- 2 198 833
- EP-A1- 2 198 841
- EP-A2- 2 198 838
- WO-A-03/084495
- WO-A1-2010/070243
- DE-A1- 19 962 869
- FR-A- 2 803 196
- FR-A- 2 919 499
- JP-A- 2003 055 174
- JP-A- 2003 081 792
- US-B1- 6 238 653

## Description

La présente invention a pour objet une composition de coloration des fibres kératiniques humaines comprenant, outre l'agent oxydant, une teneur élevée en corps gras et au moins un tensioactif non ionique polyoxyéthyléné particulier.

L'invention concerne également un procédé de coloration la mettant en oeuvre.

Parmi les méthodes de coloration des fibres kératiniques humaines, telles que les cheveux, on peut citer la coloration d'oxydation ou permanente. Plus particulièrement, ce mode de coloration met en oeuvre un ou plusieurs colorants d'oxydation, habituellement une ou plusieurs bases d'oxydation éventuellement associées à un ou plusieurs coupleurs.

En général, des bases d'oxydation sont choisies parmi les ortho- ou paraphénylènediamines, les ortho- ou para-aminophénols ainsi que des composés hétérocycliques. Ces bases d'oxydation sont des composés incolores ou faiblement colorés qui, associés à des produits oxydants, permettent d'accéder à des espèces colorées.

Bien souvent, on fait varier les nuances obtenues avec ces bases d'oxydation en les associant à un ou plusieurs coupleurs, ces derniers étant choisis notamment parmi les méta-diamines aromatiques, les méta-aminophénols, les méta-diphénols et certains composés hétérocycliques, tels que des composés indoliques.

La variété des molécules mises en jeu au niveau des bases d'oxydation et des coupleurs permet l'obtention d'une riche palette de couleurs.

Il est également possible d'ajouter à ces compositions, des colorants directs, qui sont des molécules colorées et colorantes ayant une affinité pour les fibres. Les colorants directs généralement employés sont choisis parmi les colorants directs nitrés benzéniques, anthraquinoniques, nitropyridiniques, azoïques, méthiniques, azométhiniques, xanthéniques, acridiniques, aziniques ou triarylméthaniques. La présence de tels composés permet d'enrichir encore la coloration obtenue, en reflets ou bien d'augmenter la chromaticité de la coloration obtenue.

Les procédés de coloration d'oxydation consistent donc à employer avec ces compositions tinctoriales, une composition comprenant au moins un agent oxydant, en général le peroxyde d'hydrogène, en condition de pH alcalin dans la grande majorité des cas. Cet agent oxydant a pour rôle de révéler la coloration, par une réaction de condensation oxydative des colorants d'oxydation entre eux.

La coloration d'oxydation, doit par ailleurs satisfaire un certain nombre d'exigences. Ainsi, elle doit être sans inconvénient sur le plan toxicologique, elle doit permettre d'obtenir des nuances dans l'intensité souhaitée et présenter une bonne tenue face aux agressions extérieures telles que la lumière, les intempéries, le lavage, les ondulations permanentes, la transpiration et les frottements.

Les colorants doivent également permettre de couvrir les cheveux blancs, et être enfin les moins sélectifs possibles, c'est-à-dire permettre d'obtenir des écarts de coloration les plus faibles possibles tout au long d'une même fibre kératinique, qui comporte en général des zones différemment sensibilisées (c'est-à-dire abîmées) de sa pointe à sa racine.

Les compositions obtenues doivent, en outre, présenter de bonnes propriétés de mélange et d'application, et notamment de bonnes propriétés rhéologiques pour ne pas couler, lors de leur application, sur le visage, le cuir chevelu, ou en dehors des zones que l'on se propose de teindre.

De nombreuses tentatives ont été faites dans le domaine de la coloration capillaire afin d'améliorer les propriétés tinctoriales, à l'aide, par exemple, d'adjuvants. Cependant, le choix de ces adjuvants est délicat dans la mesure où ils doivent améliorer les propriétés tinctoriales des compositions tinctoriales sans nuire aux autres propriétés de ces compositions. En particulier, ces adjuvants ne doivent pas nuire aux propriétés d'éclaircissement des fibres kératiniques et aux propriétés d'application de la coloration.

Le document JP2003-81792 décrit des compositions de colorations et de décoloration des fibres kératiniques comprenant de l'eau, un agent oxydant, des corps gras, un tensioactif, un agent alcalinisant et un agent colorant.

Le document JP2003-055174 décrit des compositions de coloration des fibres kératiniques comprenant une composition colorante alcaline et une composition oxydante comprenant un corps gras et des agents tensioactifs non ioniques.

L'un des buts de la présente invention est d'obtenir des compositions pour la coloration d'oxydation des fibres kératiniques qui ne présentent pas les inconvénients de l'art antérieur.

Plus particulièrement, l'un des buts de la présente invention est d'obtenir des compositions de coloration d'oxydation, des fibres kératiniques, présentant des propriétés tinctoriales améliorées qui permettent d'atteindre l'éclaircissement souhaité et qui soient faciles à mélanger et à appliquer, notamment qui ne coulent pas et restent bien localisées au point d'application. Par propriétés tinctoriales améliorées, on entend en particulier une amélioration au niveau de la puissance/intensité et/ou de l'homogénéité de la teinture.

Ces buts et d'autres sont atteints par la présente invention qui a pour objet une composition de coloration de fibres kératiniques humaines telle que définie dans la revendication 1.

Elle concerne également un procédé de coloration des fibres kératiniques humaines consistant à mettre en oeuvre la composition précitée.

D'autres caractéristiques et avantages de l'invention apparaîtront plus clairement à la lecture de la description et des exemples qui suivent.

Dans ce qui va suivre, et à moins d'une autre indication, les bornes d'un domaine de valeurs sont comprises dans ce domaine.

Les fibres kératiniques humaines traitées par le procédé selon l'invention sont de préférence les cheveux.

La composition de coloration selon l'invention comprend au moins 25% en poids d'un ou plusieurs corps gras différents des acides gras.

Par corps gras, on entend, un composé organique insoluble dans l'eau à température ordinaire (25°C) et à pression atmosphérique (760 mm de Hg) (solubilité inférieure à 5% et de préférence à 1% encore plus préférentiellement à 0,1%). Ils présentent dans leur structure au moins une chaîne hydrocarbonée comportant au moins 6 atomes de carbone ou un enchaînement d'au moins deux groupements siloxane. En outre, les corps gras sont généralement solubles dans des solvants organiques dans les mêmes conditions de température et de pression, comme par exemple le chloroforme, l'éthanol le benzène, l'huile de vaseline ou le décaméthylcyclopentasiloxane.

Selon l'invention, les corps gras sont choisis parmi les composés liquides ou pâteux à température ambiante et à pression atmosphérique.

Plus particulièrement, le ou les corps gras sont choisis parmi les alcanes inférieurs en C₆-C₁₆, les huiles non siliconées d'origine animale, végétale minérale ou synthétique, les alcools gras, les esters d'acide gras et/ou d'alcool gras, les cires non siliconées, les silicones.

Il est rappelé qu'au sens de l'invention, les alcools, esters et acides gras présentent plus particulièrement au moins un groupement hydrocarboné, linéaire ou ramifié, saturé ou insaturé, comprenant 6 à 30 atomes de carbone, éventuellement substitué, en particulier par un ou plusieurs groupements hydroxyle (en particulier 1 à 4). S'ils sont insaturés, ces composés peuvent comprendre une à trois double-liaisons carbone-carbone, conjuguées ou non.

En ce qui concerne les alcanes inférieurs en C₆-C₁₆, ces derniers sont linéaires, ramifiés, éventuellement cycliques. A titre d'exemple, on peut citer l'hexane, l'undécane, le dodécane, le tridécane, les isoparaffines comme l'isohexadécane, l'isodécane.

Comme huiles d'origine animale, végétale, minérale ou synthétique, utilisables dans la composition de l'invention, on peut citer par exemple :
- les huiles hydrocarbonées d'origine animale, telles que le perhydrosqualène.
- les huiles triglycérides d'origine végétale ou synthétique, telles que les triglycérides liquides d'acides gras comportant de 6 à 30 atomes de carbone comme les triglycérides des acides heptanoïque ou octanoïque ou encore, par exemple les huiles de tournesol, de maïs, de soja, de courge, de pépins de raisin, de sésame, de noisette, d'abricot, de macadamia, d'arara, de tournesol, de ricin, d'avocat, les triglycérides des acides caprylique/caprique comme ceux vendus par la société Stearineries Dubois ou ceux vendus sous les dénominations Miglyol® 810, 812 et 818 par la société Dynamit Nobel, l'huile de jojoba, l'huile de beurre de karité.
- les hydrocarbures linéaires ou ramifiés, d'origine minérale ou synthétique, de plus de 16 atomes de carbone, tels que les huiles de paraffine, volatiles ou non, et leurs dérivés, la vaseline, l'huile de vaseline, les polydécènes, les polyisobutènes hydrogénés tels que Parléam® ; de préférence les huiles de paraffine, la vaseline, l'huile de vaseline, les polydécènes, les polyisobutènes hydrogénés tels que Parléam®.
- les huiles fluorées comme le perfluorométhylcyclopentane et le perfluoro-1,3 diméthylcyclohexane, vendus sous les dénominations de "FLUTEC® PC1" et "FLUTEC® PC3" par la Société BNFL Fluorochemicals ; le perfluoro-1,2-diméthylcyclobutane ; les perfluoroalcanes tels que le dodécafluoropentane et le tétradécafluorohexane, vendus sous les dénominations de "PF 5050®" et "PF 5060®" par la Société 3M, ou encore le bromoperfluorooctyle vendu sous la dénomination "FORALKYL®" par la Société Atochem ; le nonafluoro-méthoxybutane et le nonafluoroéthoxyisobutane ; les dérivés de perfluoromorpholine, tels que la 4-trifluorométhyl perfluoromorpholine vendue sous la dénomination "PF 5052®" par la Société 3M.

Les alcools gras convenant à la mise en oeuvre de l'invention sont plus particulièrement choisis parmi les alcools saturés ou insaturés, linéaires ou ramifiés, comportant de 8 à 30 atomes de carbone. On peut citer par exemple l'alcool cétylique, l'alcool stéarylique et leur mélange (alcool cétylstéarylique), l'octyldodécanol, le 2-butyloctanol, le 2-hexyldécanol, le 2-undécylpentadécanol, l'alcool oléique ou l'alcool linoléique.

A titre d'esters d'acide gras et/ou d'alcools gras, avantageusement différents des triglycérides mentionnés ci-dessus, on peut citer notamment les esters de mono ou polyacides aliphatiques saturés ou insaturés, linéaires ou ramifiés en C₁-C₂₆ et de mono ou polyalcools aliphatiques saturés ou insaturés, linéaires ou ramifiés en C₁-C₂₆, le nombre total de carbone des esters étant supérieur ou égal à 10.

Parmi les monoesters, on peut citer le béhénate de dihydroabiétyle ; le béhénate d'octyldodécyle ; le béhénate d'isocétyle ; le lactate de cétyle ; le lactate d'alkyle en C₁₂-C₁₅ ; le lactate d'isostéaryle ; le lactate de lauryle ; le lactate de linoléyle ; le lactate d'oléyle ; l'octanoate de (iso)stéaryle ; l'octanoate d'isocétyle ; l'octanoate d'octyle ; l'octanoate de cétyle ; l'oléate de décyle ; l'isostéarate d'isocétyle ; le laurate d'isocétyle ; le stéarate d'isocétyle ; l'octanoate d'isodécyle ; l'oléate d'isodécyle ; l'isononanoate d'isononyle ; le palmitate d'isostéaryle ; le ricinoléate de méthyle acétyle ; le stéarate de myristyle ; l'isononanoate d'octyle ; l'isononate de 2-éthylhexyle ; le palmitate d'octyle ; le pélargonate d'octyle ; le stéarate d'octyle ; l'érucate d'octyldodécyle ; l'érucate d'oléyle ; les palmitates d'éthyle et d'isopropyle, le palmitate d'éthyl-2-héxyle, le palmitate de 2-octyldécyle, les myristates d'alkyles tels que le myristate d'isopropyle, de butyle, de cétyle, de 2-octyldodécyle, de mirystyle, de stéaryle le stéarate d'hexyle, le stéarate de butyle, le stéarate d'isobutyle ; le malate de dioctyle, le laurate d'hexyle, le laurate de 2-hexyldécyle.

Toujours dans le cadre de cette variante, on peut également utiliser les esters d'acides di ou tricarboxyliques en C₄-C₂₂ et d'alcools en C₁-C₂₂ et les esters d'acides mono di ou tricarboxyliques et d'alcools di, tri, tétra ou pentahydroxy en C₂-C₂₆.

On peut notamment citer : le sébacate de diéthyle ; le sébacate de diisopropyle ; l'adipate de diisopropyle ; l'adipate de di n-propyle ; l'adipate de dioctyle ; l'adipate de diisostéaryle ; le maléate de dioctyle ; l'undecylénate de glycéryle ; le stéarate d'octyldodécyl stéaroyl ; le monoricinoléate de pentaérythrityle ; le tétraisononanoate de pentaérythrityle ; le tétrapélargonate de pentaérythrityle ; le tétraisostéarate de pentaérythrityle ; le tétraoctanoate de pentaérythrityle ; le dicaprylate de propylène glycol ; le dicaprate de propylène glycol, l'érucate de tridécyle ; le citrate de triisopropyle ; le citrate de triisotéaryle ; trilactate de glycéryle ; trioctanoate de glycéryle ; le citrate de trioctyldodécyle ; le citrate de trioléyle, le dioctanoate de propylène glycol ; le diheptanoate de néopentyl glycol ; le diisanonate de diéthylène glycol ; et les distéarates de polyéthylène glycol.

Parmi les esters cités ci-dessus, on préfère utiliser les palmitates d'éthyle, d'isopropyle, de myristyle, de cétyle, de stéaryle, le palmitate d'éthyl-2-héxyle, le palmitate de 2-octyldécyle, les myristates d'alkyles tels que le myristate d'isopropyle, de butyle, de cétyle, de 2-octyldodécyle, le stéarate d'hexyle, le stéarate de butyle, le stéarate d'isobutyle ; le malate de dioctyle, le laurate d'hexyle, le laurate de 2-hexyldécyle et l'isononanate d'isononyle, l'octanoate de cétyle.

La composition peut également comprendre, à titre d'ester gras, des esters et di-esters de sucres d'acides gras en C₆-C₃₀, de préférence en C₁₂-C₂₂. Il est rappelé que l'on entend par « sucre », des composés hydrocarbonés oxygénés qui possèdent plusieurs fonctions alcool, avec ou sans fonction aldéhyde ou cétone, et qui comportent au moins 4 atomes de carbone. Ces sucres peuvent être des monosaccharides, des oligosaccharides ou des polysaccharides.

Comme sucres convenables, on peut citer par exemple le sucrose (ou saccharose), le glucose, le galactose, le ribose, le fucose, le maltose, le fructose, le mannose, l'arabinose, le xylose, le lactose, et leurs dérivés notamment alkylés, tels que les dérivés méthylés comme le méthylglucose.

Les esters de sucres et d'acides gras peuvent être choisis notamment dans le groupe comprenant les esters ou mélanges d'esters de sucres décrits auparavant et d'acides gras en C₆-C₃₀, de préférence en C₁₂-C₂₂, linéaires ou ramifiés, saturés ou insaturés. S'ils sont insaturés, ces composés peuvent comprendre une à trois double-liaisons carbone-carbone, conjuguées ou non.

Les esters selon cette variante peuvent être également choisis parmi les mono-, di-, tri- et tétra-esters, les polyesters et leurs mélanges.

Ces esters peuvent être par exemple des oléate, laurate, palmitate, myristate, béhénate, cocoate, stéarate, linoléate, linolénate, caprate, arachidonates, ou leurs mélanges comme notamment les esters mixtes oléo-palmitate, oléo-stéarate, palmito-stéarate.

Plus particulièrement, on utilise les mono- et di- esters et notamment les mono- ou di- oléate, stéarate, béhénate, oléopalmitate, linoléate, linolénate, oléostéarate, de saccharose, de glucose ou de méthylglucose.

On peut citer à titre d'exemple le produit vendu sous la dénomination Glucate® DO par la société Amerchol, qui est un dioléate de méthylglucose.

On peut aussi citer à titre d'exemples d'esters ou de mélanges d'esters de sucre d'acide gras :
- les produits vendus sous les dénominations F160, F140, F110, F90, F70, SL40 par la société Crodesta, désignant respectivement les palmito-stéarates de sucrose formés de 73 % de monoester et 27 % de di- et tri-ester, de 61 % de monoester et 39 % de di-, tri-, et tétra-ester, de 52 % de monoester et 48 % de di-, tri-, et tétra-ester, de 45 % de monoester et 55 % de di-, tri-, et tétra-ester, de 39 % de monoester et 61 % de di-, tri-, et tétra-ester, et le mono-laurate de sucrose;
- les produits vendus sous la dénomination Ryoto Sugar Esters par exemple référencés B370 et correspondant au béhénate de saccharose formé de 20 % de monoester et 80 % de di-triester-polyester;
- le mono-di-palmito-stéarate de sucrose commercialisé par la société Goldschmidt sous la dénomination Tegosoft® PSE.

La cire ou les cires non siliconées sont choisies notamment parmi la cire de Carnauba, la cire de Candelila, et la cire d'Alfa, la cire de paraffine, l'ozokérite, les cires végétales comme la cire d'olivier, la cire de riz, la cire de jojoba hydrogénée ou les cires absolues de fleurs telles que la cire essentielle de fleur de cassis vendue par la société BERTIN (France), les cires animales comme les cires d'abeilles, ou les cires d'abeilles modifiées (cerabellina) ; d'autres cires ou matières premières cireuses utilisables selon l'invention sont notamment les cires marines telles que celle vendue par la Société SOPHIM sous la référence M82, les cires de polyéthylène ou de polyoléfines en général.

Les silicones utilisables dans les compositions cosmétiques de la présente invention, sont des silicones volatiles ou non volatiles, cycliques, linéaires ou ramifiées, modifiées ou non par des groupements organiques, ayant une viscosité de 5.10⁻⁶ à 2,5m²/s à 25°C et de préférence 1.10⁻⁵ à 1m²/s.

Les silicones utilisables conformément à l'invention peuvent se présenter sous forme d'huiles, de cires, de résines ou de gommes.

De préférence, la silicone est choisie parmi les polydialkylsiloxanes, notamment les polydiméthylsiloxanes (PDMS), et les polysiloxanes organo-modifiés comportant au moins un groupement fonctionnel choisi parmi les groupements poly(oxyalkylène), les groupements aminés et les groupements alcoxy.

Les organopolysiloxanes sont définis plus en détail dans l'ouvrage de Walter NOLL "Chemistry and Technology of Silicones" (1968), Academie Press. Elles peuvent être volatiles ou non volatiles.

Lorsqu'elles sont volatiles, les silicones sont plus particulièrement choisies parmi celles possédant un point d'ébullition compris entre 60°C et 260°C, et plus particulièrement encore parmi:
(i) les polydialkylsiloxanes cycliques comportant de 3 à 7, de préférence de 4 à 5 atomes de silicium. Il s'agit, par exemple, de l'octaméthylcyclotétrasiloxane commercialisé notamment sous le nom de VOLATILE SILICONE® 7207 par UNION CARBIDE ou SILBIONE® 70045 V2 par RHODIA, le décaméthylcyclopentasiloxane commercialisé sous le nom de VOLATILE SILICONE® 7158 par UNION CARBIDE, et SILBIONE® 70045 V5 par RHODIA, ainsi que leurs mélanges.
   On peut également citer les cyclocopolymères du type diméthylsiloxanes/ méthylalkylsiloxane, tel que la SILICONE VOLATILE® FZ 3109 commercialisée par la société UNION CARBIDE, de formule : 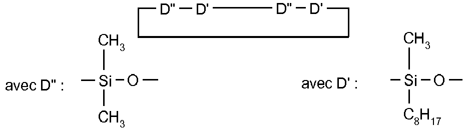
   On peut également citer les mélanges de polydialkylsiloxanes cycliques avec des composés organiques dérivés du silicium, tels que le mélange d'octaméthylcyclotétrasiloxane et de tétratriméthylsilylpentaérythritol (50/50) et le mélange d'octaméthylcyclotétrasiloxane et d'oxy-1,1'-(hexa-2,2,2',2',3,3'-triméthylsilyloxy) bis-néopentane ;
(ii) les polydialkylsiloxanes volatiles linéaires ayant 2 à 9 atomes de silicium et présentant une viscosité inférieure ou égale à 5.10⁻⁶m²/s à 25° C. Il s'agit, par exemple, du décaméthyltétrasiloxane commercialisé notamment sous la dénomination "SH 200" par la société TORAY SILICONE. Des silicones entrant dans cette classe sont également décrites dans l'article publié dans Cosmetics and Toiletries, Vol. 91, Jan. 76, P. 27-32 - TODD & BYERS "Volatile Silicone fluids for cosmetics".

On utilise de préférence des polydialkylsiloxanes non volatiles, des gommes et des résines de polydialkylsiloxanes, des polyorganosiloxanes modifiés par les groupements organofonctionnels ci-dessus ainsi que leurs mélanges.

Ces silicones sont plus particulièrement choisies parmi les polydialkylsiloxanes parmi lesquels on peut citer principalement les polydiméthylsiloxanes à groupements terminaux triméthylsilyl. La viscosité des silicones est mesurée à 25°C selon la norme ASTM 445 Appendice C.

Parmi ces polydialkylsiloxanes, on peut citer à titre non limitatif les produits commerciaux suivants :
- les huiles SILBIONE® des séries 47 et 70 047 ou les huiles MIRASIL® commercialisées par RHODIA telles que, par exemple l'huile 70 047 V 500 000 ;
- les huiles de la série MIRASIL® commercialisées par la société RHODIA ;
- les huiles de la série 200 de la société DOW CORNING telles que la DC200 ayant viscosité 60 000 mm²/s ;
- les huiles VISCASIL® de GENERAL ELECTRIC et certaines huiles des séries SF (SF 96, SF 18) de GENERAL ELECTRIC.

On peut également citer les polydiméthylsiloxanes à groupements terminaux diméthylsilanol connus sous le nom de dimethiconol (CTFA), tels que les huiles de la série 48 de la société RHODIA.

Dans cette classe de polydialkylsiloxanes, on peut également citer les produits commercialisés sous les dénominations "ABIL WAX® 9800 et 9801" par la société GOLDSCHMIDT qui sont des polydialkyl (C₁-C₂₀) siloxanes.

Les gommes de silicone utilisables conformément à l'invention sont notamment des polydialkylsiloxanes, de préférence des polydiméthylsiloxanes ayant des masses moléculaires moyennes en nombre élevées comprises entre 200 000 et 1 000 000 utilisés seuls ou en mélange dans un solvant. Ce solvant peut être choisi parmi les silicones volatiles, les huiles polydiméthylsiloxanes (PDMS), les huiles polyphénylméthylsiloxanes (PPMS), les isoparaffines, les polyisobutylènes, le chlorure de méthylène, le pentane, le dodécane, le tridécane ou leurs mélanges.

Des produits plus particulièrement utilisables conformément à l'invention sont des mélanges tels que :
- les mélanges formés à partir d'un polydiméthylsiloxane hydroxylé en bout de chaîne, ou diméthiconol (CTFA) et d'un polydiméthylsiloxane cyclique également appelé cyclométhicone (CTFA) tel que le produit Q2 1401 commercialisé par la société DOW CORNING ;
- les mélanges d'une gomme polydiméthylsiloxane et d'une silicone cyclique tel que le produit SF 1214 Silicone Fluid de la société GENERAL ELECTRIC, ce produit est une gomme SF 30 correspondant à une diméthicone, ayant un poids moléculaire moyen en nombre de 500 000 solubilisée dans l'huile SF 1202 Silicone Fluid correspondant au décaméthylcyclopentasiloxane ;
- les mélanges de deux PDMS de viscosités différentes, et plus particulièrement d'une gomme PDMS et d'une huile PDMS, tels que le produit SF 1236 de la société GENERAL ELECTRIC. Le produit SF 1236 est le mélange d'une gomme SE 30 définie ci-dessus ayant une viscosité de 20 m²/s et d'une huile SF 96 d'une viscosité de 5.10⁻⁶m²/s. Ce produit comporte de préférence 15 % de gomme SE 30 et 85 % d'une huile SF 96.

Les résines d'organopolysiloxanes utilisables conformément à l'invention sont des systèmes siloxaniques réticulés renfermant les motifs :

R₂SiO_{2/2}, R₃SiO_{1/2}, RSiO_{3/2} et SiO_{4/2}

dans lesquelles R représente un alkyl possédant 1 à 16 atomes de carbone. Parmi ces produits, ceux particulièrement préférés sont ceux dans lesquels R désigne un groupe alkyle inférieur en C₁-C₄, plus particulièrement méthyle.

On peut citer parmi ces résines le produit commercialisé sous la dénomination "DOW CORNING 593" ou ceux commercialisés sous les dénominations "SILICONE FLUID SS 4230 et SS 4267" par la société GENERAL ELECTRIC et qui sont des silicones de structure diméthyl/triméthyl siloxane.

On peut également citer les résines du type triméthylsiloxysilicate commercialisées notamment sous les dénominations X22-4914, X21-5034 et X21-5037 par la société SHIN-ETSU.

Les silicones organomodifiées utilisables conformément à l'invention sont des silicones telles que définies précédemment et comportant dans leur structure un ou plusieurs groupements organofonctionnels fixés par l'intermédiaire d'un groupe hydrocarboné.

Outre, les silicones décrites ci-dessus les silicones organomodifiées peuvent être des polydiaryl siloxanes, notamment des polydiphénylsiloxanes, et des polyalkylarylsiloxanes fonctionnalisés par les groupes organofonctionnels mentionnés précédemment.

Les polyalkylarylsiloxanes sont particulièrement choisis parmi les polydiméthyl/méthylphénylsiloxanes, les polydiméthyl/diphénylsiloxanes linéaires et/ou ramifiés de viscosité allant de 1.10⁻⁵ à 5.10⁻²m²/s à 25°C.

Parmi ces polyalkylarylsiloxanes on peut citer à titre d'exemple les produits commercialisés sous les dénominations suivantes :
- les huiles SILBIONE® de la série 70 641 de RHODIA;
- les huiles des séries RHODORSIL® 70 633 et 763 de RHODIA ;
- l'huile DOW CORNING 556 COSMETIC GRAD FLUID de DOW CORNING ;
- les silicones de la série PK de BAYER comme le produit PK20 ;
- les silicones des séries PN, PH de BAYER comme les produits PN1000 et PH1000 ;
- certaines huiles des séries SF de GENERAL ELECTRIC telles que SF 1023, SF 1154, SF 1250, SF 1265.

Parmi les silicones organomodifiées, on peut citer les polyorganosiloxanes comportant :
- des groupements polyéthylèneoxy et/ou polypropylèneoxy comportant éventuellement des groupements alkyle en C₆-C₂₄ tels que les produits dénommés diméthicone copolyol commercialisé par la société DOW CORNING sous la dénomination DC 1248 ou les huiles SILWET® L 722, L 7500, L 77, L 711 de la société UNION CARBIDE et l'alkyl (C₁₂)-méthicone copolyol commercialisée par la société DOW CORNING sous la dénomination Q2 5200 ;
- des groupements aminés substitués ou non comme les produits commercialisés sous la dénomination GP 4 Silicone Fluid et GP 7100 par la société GENESEE ou les produits commercialisés sous les dénominations Q2 8220 et DOW CORNING 929 ou 939 par la société DOW CORNING. Les groupements aminés substitués sont en particulier des groupements aminoalkyle en C₁-C₄ ;
- des groupements alcoxylés, comme le produit commercialisé sous la dénomination "SILICONE COPOLYMER F-755" par SWS SILICONES et ABIL WAX® 2428, 2434 et 2440 par la société GOLDSCHMIDT.

De préférence, le ou les corps gras ne comprennent pas de motif oxyalkyléné en C₂-C₃ ni de motif glycérolé.

De préférence, le corps gras est un composé liquide à la température de 25°C et à la pression atmosphérique.

Les corps gras sont de préférence choisis parmi les alcanes inférieurs en C₆-C₁₆, les huiles non siliconées d'origine végétale, minérales ou synthétique, les alcools gras, les esters d'acide gras et/ou d'alcool gras, les silicones, ou leurs mélanges.

De préférence, le corps gras est choisi parmi l'huile de vaseline, les polydécènes, les esters d'acide gras et/ou d'alcool gras liquides, ou leurs mélanges.

Comme indiqué auparavant, la composition selon l'invention comprend au moins 25% de corps gras. La composition selon l'invention présente plus particulièrement une teneur en corps gras allant de 25 à 80% en poids, encore plus préférentiellement de 25 à 65%, mieux de 30 à 55% en poids par rapport au poids de la composition.

La composition selon l'invention comprend en outre de 1 à 10% en poids par rapport au poids de la composition, d'un ou plusieurs tensioactifs non ioniques polyoxyéthylénés comprenant au moins 10 moles d'oxyde d'éthylène et au plus 80 moles d'oxyde d'éthylène.

A titre d'exemples de tensioactifs non ioniques oxyéthylénés convenables, on peut citer, seuls ou en mélanges :
- les alkyl(C₈-C₂₄)phénols oxyéthylénés,
- les alcools en C₈-C₃₀, saturés ou non, linéaires ou ramifiés, oxyéthylénés,
- les amides, en C₈-C₃₀, saturés ou non, linéaires ou ramifiés, oxyéthylénés,
- les esters d'acides en C₈-C₃₀, saturés ou non, linéaires ou ramifiés, et de polyéthylèneglycols,
- les esters d'acides en C₈-C₃₀, saturés ou non, linéaires ou ramifiés, et de sorbitol polyoxyéthylénés,
- les huiles végétales oxyéthylénées, saturées ou non ;
le nombre de moles d'oxyde d'éthylène varie de 10 à moins de 80, et de préférence.

La teneur en tensioactifs non ioniques oxyéthylénés est de manière plus avantageuse comprise entre 1 et 8% en poids par rapport au poids de la composition selon l'invention.

La composition selon l'invention comprend un ou plusieurs colorants choisis parmi les colorants d'oxydation qui seront détaillés ci-dessous.

Les colorants d'oxydation sont en général choisis parmi une ou plusieurs bases d'oxydation éventuellement combinée(s) à un ou plusieurs coupleurs.

A titre d'exemple, les bases d'oxydation sont choisies parmi les paraphénylènediamines, les bis-phénylalkylènediamines, les para-aminophénols, les ortho-aminophénols, les bases hétérocycliques et leurs sels d'addition.

Parmi les paraphénylènediamines, on peut citer à titre d'exemple, la paraphénylènediamine, la paratoluylènediamine, la 2-chloro paraphénylènediamine, la 2,3-diméthyl paraphénylènediamine, la 2,6-diméthyl paraphénylènediamine, la 2,6-diéthyl paraphénylènediamine, la 2,5-diméthyl paraphénylènediamine, la N,N-diméthyl paraphénylènediamine, la N,N-diéthyl paraphénylènediamine, la N,N-dipropyl paraphénylènediamine, la 4-amino N,N-diéthyl 3-méthyl aniline, la N,N-bis-(β-hydroxyéthyl) paraphénylènediamine, la 4-N,N-bis-(β-hydroxyéthyl)amino 2-méthyl aniline, la 4-N,N-bis-(P-hydroxyéthyl)amino 2-chloro aniline, la 2-β-hydroxyéthyl paraphénylènediamine, la 2-fluoro paraphénylènediamine, la 2-isopropyl paraphénylènediamine, la N-(β-hydroxypropyl) paraphénylènediamine, la 2-hydroxyméthyl paraphénylènediamine, la N,N-diméthyl 3-méthyl paraphénylènediamine, la N,N-(éthyl, β-hydroxyéthyl) paraphénylènediamine, la N-(β,γ-dihydroxypropyl) paraphénylènediamine, la N-(4'-aminophényl) paraphénylènediamine, la N-phényl paraphénylènediamine, la 2-β-hydroxyéthyloxy paraphénylènediamine, la 2-β-acétylaminoéthyloxy paraphénylènediamine, la N-(β-méthoxyéthyl) paraphénylène-diamine, la 4-aminophénylpyrrolidine, la 2-thiényl paraphénylènediamine, le 2-β hydroxyéthylamino 5-amino toluène, la 3-hydroxy 1-(4'-aminophényl)pyrrolidine et leurs sels d'addition avec un acide.

Parmi les paraphénylènediamines citées ci-dessus, la paraphénylènediamine, la paratoluylènediamine, la 2-isopropyl paraphénylènediamine, la 2-β-hydroxyéthyl paraphénylènediamine, la 2-β-hydroxyéthyloxy paraphénylène-diamine, la 2,6-diméthyl paraphénylènediamine, la 2,6-diéthyl paraphénylènediamine, la 2,3-diméthyl paraphénylènediamine, la N,N-bis-(β-hydroxyéthyl) paraphénylènediamine, la 2-chloro paraphénylènediamine, la 2-β-acétylaminoéthyloxy paraphénylènediamine, et leurs sels d'addition avec un acide sont particulièrement préférées.

Parmi les bis-phénylalkylènediamines, on peut citer à titre d'exemple, le N,N'-bis-(β-hydroxyéthyl) N,N'-bis-(4'-aminophényl) 1,3-diamino propanol, la N,N'-bis-(β-hydroxyéthyl) N,N'-bis-(4'-aminophényl) éthylènediamine, la N,N'-bis-(4-aminophényl) tétraméthylènediamine, la N,N'-bis-(β-hydroxyéthyl) N,N'-bis-(4-aminophényl) tétraméthylènediamine, la N,N'-bis-(4-méthyl-aminophényl) tétraméthylènediamine, la N,N'-bis-(éthyl) N,N'-bis-(4'-amino, 3'-méthylphényl) éthylènediamine, le 1,8-bis-(2,5-diamino phénoxy)-3,6-dioxaoctane, et leurs sels d'addition.

Parmi les para-aminophénols, on peut citer à titre d'exemple, le para-aminophénol, le 4-amino 3-méthyl phénol, le 4-amino 3-fluoro phénol, le 4-amino-3-chlorophénol, le 4-amino 3-hydroxyméthyl phénol, le 4-amino 2-méthyl phénol, le 4-amino 2-hydroxyméthyl phénol, le 4-amino 2-méthoxyméthyl phénol, le 4-amino 2-aminométhyl phénol, le 4-amino 2-(β-hydroxyéthyl aminométhyl) phénol, le 4-amino 2-fluoro phénol, et leurs sels d'addition avec un acide.

Parmi les ortho-aminophénols, on peut citer à titre d'exemple, le 2-amino phénol, le 2-amino 5-méthyl phénol, le 2-amino 6-méthyl phénol, le 5-acétamido 2-amino phénol, et leurs sels d'addition.

Parmi les bases hétérocycliques, on peut citer à titre d'exemple, les dérivés pyridiniques, les dérivés pyrimidiniques et les dérivés pyrazoliques.

Parmi les dérivés pyridiniques, on peut citer les composés décrits par exemple dans les brevets GB 1 026 978 et GB 1 153 196, comme la 2,5-diamino pyridine, la 2-(4-méthoxyphényl)amino 3-amino pyridine, la 3,4-diamino pyridine, et leurs sels d'addition.

D'autres bases d'oxydation pyridiniques utiles dans la présente invention sont les bases d'oxydation 3-amino pyrazolo-[1,5-a]-pyridines ou leurs sels d'addition décrits par exemple dans la demande de brevet FR 2801308. A titre d'exemple, on peut citer la pyrazolo[1,5-a]pyridin-3-ylamine ; la 2-acétylamino pyrazolo-[1,5-a] pyridin-3-ylamine ; la 2-morpholin-4-yl-pyrazolo[1,5-a]pyridin-3-ylamine ; l'acide 3-amino-pyrazolo[1,5-a]pyridin-2-carboxylique ; la 2-méthoxy-pyrazolo[1,5-a]pyridine-3-ylamino ; le (3-amino-pyrazolo[1,5-a]pyridine-7-yl)-méthanol ; le 2-(3-amino-pyrazolo[1,5-a]pyridine-5-yl)-éthanol ; le 2-(3-amino-pyrazolo[1,5-a]pyridine-7-yl)-éthanol ; le (3-amino-pyrazolo[1,5-a]pyridine-2-yl)-méthanol ; la 3,6-diamino-pyrazolo[1,5-a]pyridine ; la 3,4-diamino-pyrazolo[1,5-a]pyridine ; la pyrazolo[1,5-a]pyridine-3,7-diamine ; la 7-morpholin-4-yl-pyrazolo[1,5-a]pyridin-3-ylamine ; la pyrazolo[1,5-a]pyridine-3,5-diamine ; la 5-morpholin-4-yl-pyrazolo[1,5-a]pyridin-3-ylamine ; le 2-[(3-amino-pyrazolo[1,5-a]pyridin-5-yl)-(2-hydroxyéthyl)-amino]-éthanol ; le 2-[(3-amino-pyrazolo[1,5-a]pyridin-7-yl)-(2-hydroxyéthyl)-amino]-éthanol ; la 3-amino-pyrazolo[1,5-a]pyridine-5-ol ; 3-amino-pyrazolo[1,5-a]pyridine-4-ol ; la 3-amino-pyrazolo[1,5-a]pyridine-6-ol ; la 3-amino-pyrazolo[1,5-a]pyridine-7-ol ; ainsi que leurs sels d'addition.

Parmi les dérivés pyrimidiniques, on peut citer les composés décrits par exemple dans les brevets DE 2359399 ; JP 88-169571 ; JP 05-63124 ; EP 0770375 ou demande de brevet WO 96/15765 comme la 2,4,5,6-tétra-aminopyrimidine, la 4-hydroxy 2,5,6-triaminopyrimidine, la 2-hydroxy 4,5,6-triaminopyrimidine, la 2,4-dihydroxy 5,6-diaminopyrimidine, la 2,5,6-triaminopyrimidine et leurs sels d'addition et leurs formes tautomères, lorsqu'il existe un équilibre tautomérique.

Parmi les dérivés pyrazoliques, on peut citer les composés décrits dans les brevets DE 3843892, DE 4133957 et demandes de brevet WO 94/08969, WO 94/08970, FR-A-2 733 749 et DE 195 43 988 comme le 4,5-diamino 1-méthyl pyrazole, le 4,5-diamino 1-(β-hydroxyéthyl) pyrazole, le 3,4-diamino pyrazole, le 4,5-diamino 1-(4'-chlorobenzyl) pyrazole, le 4,5-diamino 1,3-diméthyl pyrazole, le 4,5-diamino 3-méthyl 1-phényl pyrazole, le 4,5-diamino 1-méthyl 3-phényl pyrazole, le 4-amino 1,3-diméthyl 5-hydrazino pyrazole, le 1-benzyl 4,5-diamino 3-méthyl pyrazole, le 4,5-diamino 3-tert-butyl 1-méthyl pyrazole, le 4,5-diamino 1-tert-butyl 3-méthyl pyrazole, le 4,5-diamino 1-(β-hydroxyéthyl) 3-méthyl pyrazole, le 4,5-diamino 1-éthyl 3-méthyl pyrazole, le 4,5-diamino 1-éthyl 3-(4'-méthoxyphényl) pyrazole, le 4,5-diamino 1-éthyl 3-hydroxyméthyl pyrazole, le 4,5-diamino 3-hydroxyméthyl 1-méthyl pyrazole, le 4,5-diamino 3-hydroxyméthyl 1-isopropyl pyrazole, le 4,5-diamino 3-méthyl 1-isopropyl pyrazole, le 4-amino 5-(2'-aminoéthyl)amino 1,3-diméthyl pyrazole, le 3,4,5-triamino pyrazole, le 1-méthyl 3,4,5-triamino pyrazole, le 3,5-diamino 1-méthyl 4-méthylamino pyrazole, le 3,5-diamino 4-(β-hydroxyéthyl)amino 1-méthyl pyrazole, et leurs sels d'addition. On peut aussi utiliser le 4-5-diamino 1-(β-méthoxyéthyl)pyrazole.

De préférence, on utilisera un 4,5-diaminopyrazole et encore plus préférentiellement le 4,5-diamino-1-(β-hydroxyéthyl)-pyrazole et/ou l'un des ses sels.

A titre de dérivés pyrazoliques, on peut également citer les diamino N,N-dihydropyrazolopyrazolones et notamment celles décrites dans la demande FR-A-2 886 136 telles que les composés suivants et leurs sels d'addition : 2,3-diamino-6,7-dihydro-1H,5H-pyrazolo[1,2-a]pyrazol-1-one, 2-amino-3-éthylamino-6,7-dihydro-1H,5H-pyrazolo[1,2-a]pyrazol-1-one, 2-amino-3-isopropylamino-6,7-dihydro-1H,5H-pyrazolo[1,2-a]pyrazol-1-one, 2-amino-3-(pyrrolidin-1-yl)-6,7-dihydro-1H,5H-pyrazolo[1,2-a]pyrazol-1-one, 4,5-diamino-1,2-diméthyl-1,2-dihydro-pyrazol-3-one, 4,5-diamino-1,2-diéthyl-1,2-dihydro-pyrazol-3-one, 4,5-diamino-1,2-di-(2-hydroxyéthyl)-1,2-dihydro-pyrazol-3-one, 2-amino-3-(2-hydroxyéthyl)amino-6,7-dihydro-1H,5H-pyrazolo[1,2-a]pyrazol-1-one, 2-amino-3-diméthylamino-6,7-dihydro-1H,5H-pyrazolo[1,2-a]pyrazol-1-one, 2,3-diamino-5,6,7,8-tétrahydro-1H,6H-pyridazino[1,2-a]pyrazol-1-one, 4-amino-1,2-diéthyl-5-(pyrrolidin-1-yl)-1,2-dihydro-pyrazol-3-one, 4-amino-5-(3-diméthylamino-pyrrolidin-1-yl)-1,2-diéthyl-1,2-dihydro-pyrazol-3-one, 2,3-diamino-6-hydroxy-6,7-dihydro-1H,5H-pyrazolo[1,2-a]pyrazol-1-one.

On préférera utiliser la 2,3-diamino-6,7-dihydro-1H,5H-pyrazolo[1,2-a]pyrazol-1-one et/ou un de ses sels.

A titre de bases hétérocycliques, on utilisera préférentiellement le 4,5-diamino-1-(β-hydroxyéthyl)pyrazole et/ou la 2,3-diamino-6,7-dihydro-1H,5H-pyrazolo[1,2-a]pyrazol-1-one et/ou un de leurs sels.

La composition selon l'invention peut éventuellement comprendre un ou plusieurs coupleurs choisis avantageusement parmi ceux conventionnellement utilisés pour la teinture des fibres kératiniques.

Parmi ces coupleurs, on peut notamment citer les méta-phénylènediamines, les méta-aminophénols, les méta-diphénols, les coupleurs naphtaléniques, les coupleurs hétérocycliques ainsi que leurs sels d'addition.

A titre d'exemple, on peut citer le 1,3-dihydroxy benzène, le 1,3-dihydroxy 2-méthyl benzène, le 4-chloro 1,3-dihydroxy benzène, le 2,4-diamino 1-(β-hydroxyéthyloxy) benzène, le 2-amino 4-(β-hydroxyéthylamino) 1-méthoxybenzène, le 1,3-diamino benzène, le 1,3-bis-(2,4-diaminophénoxy) propane, la 3-uréido aniline, le 3-uréido 1-diméthylamino benzène, le sésamol, le 1-β-hydroxyéthylamino-3,4-méthylènedioxybenzène, l'α-naphtol, le 2 méthyl-1-naphtol, le 6-hydroxy indole, le 4-hydroxy indole, le 4-hydroxy N-méthyl indole, la 2-amino-3-hydroxy pyridine, la 6-hydroxy benzomorpholine la 3,5-diamino-2,6-diméthoxypyridine, le 1-N-(β-hydroxyéthyl)amino-3,4-méthylène dioxybenzène, le 2,6-bis-(β-hydroxyéthylamino)toluène, la 6-hydroxy indoline, la 2,6-dihydroxy 4-méthyl pyridine, la 1-H 3-méthyl pyrazole 5-one, la 1-phényl 3-méthyl pyrazole 5-one, le 2,6-diméthyl pyrazolo [1,5-b]-1,2,4-triazole, le 2,6-diméthyl [3,2-c]-1,2,4-triazole, le 6-méthyl pyrazolo [1,5-a]-benzimidazole, leurs sels d'addition avec un acide, et leurs mélanges.

D'une manière générale, les sels d'addition des bases d'oxydation et des coupleurs utilisables dans le cadre de l'invention sont notamment choisis parmi les sels d'addition avec un acide tels que les chlorhydrates, les bromhydrates, les sulfates, les citrates, les succinates, les tartrates, les lactates, les tosylates, les benzènesulfonates, les phosphates et les acétates.

La ou les bases d'oxydation représentent chacune avantageusement de 0,0001 à 10% en poids par rapport au poids total de la composition, et de préférence de 0,005 à 5% en poids par rapport au poids total de la composition.

La teneur en coupleur(s), s'il(s) est(sont) présent(s), représentent chacun avantageusement de 0,0001 à 10% en poids par rapport au poids total de la composition, et de préférence de 0,005 à 5 en poids par rapport au poids total de la composition.

La composition selon l'invention peut éventuellement comprendre, éventuellement en plus du ou des colorants d'oxydation et de préférence en plus du ou des colorants d'oxydation, un ou plusieurs colorants directs, synthétiques ou naturels, choisis parmi les espèces ioniques ou non ioniques, de préférence cationiques ou non ioniques.

A titre d'exemples de colorants directs convenables, on peut citer les colorants directs azoïques ; méthiniques ; carbonyles ; aziniques ; nitrés (hétéro)aryle ; tri-(hétéro)aryle méthanes ; les porphyrines ; les phtalocyanines et les colorants directs naturels, seuls ou en mélanges.

Parmi les colorants directs naturels utilisables selon l'invention, on peut citer la lawsone, la juglone, l'alizarine, la purpurine, l'acide carminique, l'acide kermésique, la purpurogalline, le protocatéchaldéhyde, l'indigo, l'isatine, la curcumine, la spinulosine, l'apigénidine, les orcéines. On peut également utiliser les extraits ou décoctions contenant ces colorants naturels et notamment les cataplasmes ou extraits à base de henné.

Lorsqu'ils sont présents, le ou les colorants directs représentent plus particulièrement de 0,0001 à 10% en poids du poids total de la composition, et de préférence de 0,005 à 5% en poids.

La composition selon l'invention comprend également un ou plusieurs agents oxydants.

Plus particulièrement, le ou les agents oxydants sont choisis parmi le peroxyde d'hydrogène, le peroxyde d'urée, les bromates ou ferricyanures de métaux alcalins, les sels peroxygénés comme par exemple les persulfates, les perborates et les percarbonates de métaux alcalins ou alcalino-terreux, ainsi que les peracides et leurs précurseurs.

De préférence, l'agent oxydant n'est pas choisi parmi les sels peroxygénés.

Avantageusement, l'agent oxydant est le peroxyde d'hydrogène.

La teneur en agent(s) oxydant(s) représente plus particulièrement de 0,1 à 20% en poids, de préférence de 0,5 à 10% en poids, par rapport au poids de la composition.

La composition selon l'invention comprend également un ou plusieurs agents alcalinisants.

L'agent alcalinisant peut être minéral ou organique ou hybride.

Le ou les agents alcalinisants minéraux sont de préférence choisis parmi l'ammoniaque, les carbonates ou bicarbonates alcalins, les hydroxydes de sodium ou de potassium ou leurs mélanges.

Le ou les, agents alcalinisants organiques sont de préférence choisis parmi les amines organiques dont le pKb à 25°C est inférieur à 12, et de préférence inférieur à 10, encore plus avantageusement inférieur à 6. Il est à noter qu'il s'agit du pKb correspondant à la fonction de basicité la plus élevée.

Le ou les agents alcalinisants organiques sont par exemple choisis parmi les alcanolamines, les éthylènediamines oxyéthylénées et/ou oxypropylénées, les acides aminés et les composés de formule (IX) suivante : 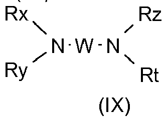 dans laquelle W est un reste alkylène en C₁-C₆ éventuellement substitué par un groupement hydroxyle ou un radical alkyle en C₁-C₆ ; Rx, Ry, Rz et Rt, identiques ou différents, représentent un atome d'hydrogène, un radical alkyle en C₁-C₆ ou hydroxyalkyle en C₁-C₆, aminoalkyle en C₁-C₆.

On peut citer à titre d'exemple de telles aminés, le 1,3 diaminopropane, le 1,3 diamino 2 propanol, la spermine, la spermidine.

Par alcanolamine on entend une amine organique comprenant une fonction amine primaire, secondaire ou tertiaire, et un ou plusieurs groupements alkyle, linéaires ou ramifiés, en C₁-C₈ porteurs d'un ou plusieurs radicaux hydroxyle.

Conviennent en particulier à la réalisation de l'invention les alcanolamines telles que les mono-, di- ou tri- alcanolamines, comprenant un à trois radicaux hydroxyalkyle, identiques ou non, en C₁-C₄.

Parmi des composés de ce type, on peut citer la monoéthanolamine, la diéthanolamine, la triéthanolamine, la monoisopropanolamine, la diisopropanolamine, la N-diméthylaminoéthanolamine, le 2-amino-2-méthyl-1-propanol, la triisopropanolamine, le 2-amino-2-méthyl-1,3-propanediol, le 3-amino-1,2-propanediol, le 3-diméthylamino-1,2-propanediol, le tris-hydroxyméthylamino-méthane.

Plus particulièrement, les acides aminés utilisables sont d'origine naturelle ou de synthèse, sous leur forme L, D, ou racémique et comportent au moins une fonction acide choisie plus particulièrement parmi les fonctions acides carboxyliques, sulfoniques, phosphoniques ou phosphoriques. Les acides aminés peuvent se trouver sous forme neutre ou ionique.

A titre d'acides aminés utilisables dans la présente invention, on peut notamment citer l'acide aspartique, l'acide glutamique, l'alanine, l'arginine, l'ornithine, la citrulline, l'asparagine, la carnitine, la cystéine, la glutamine, la glycine, l'histidine, la lysine, l'isoleucine, la leucine, la méthionine, la N-phénylalanine, la proline, la serine, la taurine la thréonine, le tryptophane, la tyrosine et la valine.

De manière avantageuse, les acides aminés sont des acides aminés basiques comprenant une fonction amine supplémentaire éventuellement incluse dans un cycle ou dans une fonction uréido.

De tels acides aminés basiques sont choisis de préférence parmi ceux répondant à la formule (X) suivante : 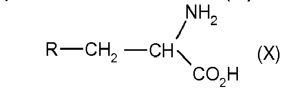 où R désigne un groupe choisi parmi :

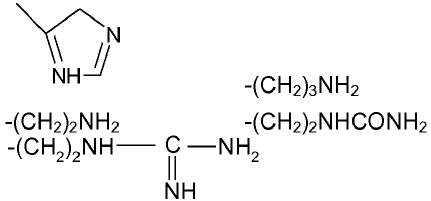

Les composés correspondants à la formule (X) sont l'histidine, la lysine, l'arginine, l'ornithine, la citrulline.

L'amine organique peut être aussi choisie parmi les amines organiques de type hétérocycliques On peut en particulier citer, outre l'histidine déjà mentionnée dans les acides aminés, la pyridine, la pipéridine, l'imidazole, le triazole, le tétrazole, le benzimidazole.

L'amine organique peut être aussi choisie parmi les dipeptides d'acides aminés. A titre de dipeptides d'acides aminés utilisables dans la présente invention, on peut notamment citer la carnosine, l'anserine et la baleine

L'amine organique peut aussi être choisie parmi les composés comportant une fonction guanidine. A titre d'amines d'amines de ce type utilisables dans la présente invention, on peut notamment citer outre l'arginine déjà mentionnée à titre d'acide aminé, la créatine, la créatinine, la 1,1-diméthylguanidine, 1,1-diéthylguanidine, la glycocyamine, la metformin, l'agmatine, la n-amidinoalanine, l'acide 3-guanidinopropionique, l'acide 4-guanidinobutyrique et l'acide 2-([amino(imino)methyl]amino)ethane-1-sulfonique.

De préférence l'amine organique présente dans la composition de l'invention est une alcanolamine.

Encore plus préférentiellement l'amine organique est la monoéthanolamine.

A titre de composés hybrides on peut mentionner les sels des amines citées précédemment avec des acides comme l'acide carbonique, l'acide chlorhydrique.

On peut en particulier utiliser le carbonate de guanidine ou le chlorhydrate de monoéthanolamine.

De manière avantageuse, la composition selon l'invention présente une teneur en agent(s) alcalinisant(s) allant de 0,01 à 30% en poids, de préférence de 0,1 à 20% en poids par rapport au poids de ladite composition.

Il est à noter que, de préférence, la composition selon l'invention ne comprend pas d'ammoniaque ou un de ses sels, en tant qu'agent alcalinisant. Si toutefois elle en comprenait, sa teneur ne dépasserait pas 0,03% en poids (exprimé en NH₃), de préférence ne dépasserait pas 0,01% en poids, par rapport au poids de la composition selon l'invention. De préférence, si la composition comprend de l'ammoniaque ou un de ses sels, alors la quantité d'agent(s) alcalinisant(s) est supérieure à celle d'ammoniaque (exprimé en NH₃).

La composition de l'invention contient de préférence une ou plusieurs alcanolamines, et/ou un ou plusieurs acides aminés basiques.

De préférence la composition de l'invention comprend de la monoéthanolamine.

La composition selon l'invention peut également contenir ou plusieurs tensioactifs additionnels.

De préférence, le ou les tensioactifs additionnels sont choisis parmi les tensioactifs non ioniques différents de ceux mentionnés auparavant, ou parmi les tensioactifs anioniques.

Les tensioactifs anioniques sont plus spécialement choisis parmi les sels (en particulier sels de métaux alcalins, notamment de sodium, sels d'ammonium, sels d'amines, sels d'aminoalcools ou sels de métaux alcalino-terreux comme le magnésium) des composés suivants :
- les alkylsulfates, les alkyléthersulfates, alkylamidoéthersulfates, alkylaryl-polyéthersulfates, monoglycérides sulfates ;
- les alkylsulfonates, alkylamidesulfonates, alkylarylsulfonates, α-oléfine-sulfonates, paraffine-sulfonates ;
- les alkylphosphates, les alkylétherphosphates;
- les alkylsulfosuccinates, les alkyléthersulfosuccinates, les alkylamide-sulfosuccinates; les alkylsulfosuccinamates ;
- les alkylsulfoacétates ;
- les acylsarcosinates ; les acyliséthionates et les N-acyltaurates ;
- les sels d'acides gras tels que les acides oléique, ricinoléique, palmitique, stéarique, les acides d'huile de coprah ou d'huile de coprah hydrogénée ;
- les sels d'acides d'alkyl D galactoside uroniques ;
- les acyl-lactylates ;
- les sels des acides alkyléther carboxyliques polyoxyalkylénés, des acides alkylaryléther carboxyliques polyoxyalkylénés, des acides alkylamidoéther carboxyliques polyoxyalkylénés, en particulier ceux comportant de 2 à 50 groupements oxyde d'éthylène ;
- et leurs mélanges.

Il est à noter que le radical alkyle ou acyle de ces différents composés comporte avantageusement de 6 à 24 atomes de carbone, et de préférence de 8 à 24 atomes de carbone, et le radical aryle désignant de préférence un groupement phényle ou benzyle.

Les tensioactifs non ioniques sont plus particulièrement choisis parmi les tensioactifs non ioniques mono ou poly- oxyalkylénés différents des tensioactifs non ioniques précités, ou encore mono- ou poly- glycérolés. Les motifs oxyalkylénés sont plus particulièrement des motifs oxyéthylénés, oxypropylénés, ou leur combinaison, de préférence oxyéthylénés.

A titre d'exemples de tensioactifs non ioniques oxyalkylénés, on peut citer :
- les alkyl(C₈-C₂₄)phénols oxyalkylénés,
- les alcools en C₈-C₃₀, saturés ou non, linéaires ou ramifiés, oxyalkylénés,
- les amides, en C₈-C₃₀, saturés ou non, linéaires ou ramifiés, oxyalkylénés,
- les esters d'acides en C₈-C₃₀, saturés ou non, linéaires ou ramifiés, et de polyéthylèneglycols,
- les esters d'acides en C₈-C₃₀, saturés ou non, linéaires ou ramifiés, et de sorbitol polyoxyéthylénés,
- les huiles végétales oxyéthylénées, saturées ou non,
- les condensats d'oxyde d'éthylène et/ou d'oxyde de propylène, entre autres, seuls ou en mélanges.

Les tensioactifs présentant un nombre de moles d'oxyde d'éthylène compris entre 1 et 9 et/ou un nombre de moles d'oxyde de propylène compris entre 1 et 50.

De manière avantageuse, les tensioactifs non ioniques ne comprennent pas de motifs oxypropylénés.

A titre d'exemple de tensioactifs non ioniques mono- ou poly- glycérolés, on utilise de préférence les alcools en C₈-C₄₀, mono- ou poly- glycérolés.

En particulier, les alcools en C₈-C₄₀ mono- ou poly- glycérolés correspondent à la formule suivante :

RO-[CH₂-CH(CH₂OH)-O]ₘ-H

dans laquelle R représente un radical alkyle ou alcényle, linéaire ou ramifié, en C₈-C₄₀, de préférence en C₈-C₃₀, et m représente un nombre allant de 1 à 30 et de préférence de 1 à 10.

A titre d'exemple de composés convenables dans le cadre de l'invention, on peut citer, l'alcool laurique à 4 moles de glycérol (nom INCI : POLYGLYCERYL-4 LAURYL ETHER), l'alcool laurique à 1,5 moles de glycérol, l'alcool oléique à 4 moles de glycérol (nom INCI : POLYGLYCERYL-4 OLEYL ETHER), l'alcool oléique à 2 moles de glycérol (Nom INCI: POLYGLYCERYL-2 OLEYL ETHER), l'alcool cétéarylique à 2 moles de glycérol, l'alcool cétéarylique à 6 moles de glycérol, l'alcool oléocétylique à 6 moles de glycérol, et l'octadécanol à 6 moles de glycérol.

L'alcool peut représenter un mélange d'alcools au même titre que la valeur de m représente une valeur statistique, ce qui signifie que dans un produit commercial peuvent coexister plusieurs espèces d'alcools gras polyglycérolés sous forme d'un mélange.

Parmi les alcools mono- ou poly-glycérolés, on préfère plus particulièrement utiliser l'alcool en C₈/C₁₀ à une mole de glycérol, l'alcool en C₁₀/C₁₂ à 1 mole de glycérol et l'alcool en C₁₂ à 1,5 mole de glycérol.

Lorsqu'ils sont présents, les tensioactifs additionnels représentent plus particulièrement de 0,1 à 50% en poids, de préférence de 0,5 à 30% en poids par rapport au poids de la composition.

La composition peut également renfermer divers adjuvants utilisés classiquement dans les compositions pour la coloration des cheveux, tels que des polymères anioniques, non ioniques, amphotères, zwitterioniques ou leurs mélanges ; des agents antioxydants ; des agents de pénétration ; des agents séquestrants ; des parfums ; des agents dispersants ; des agents filmogènes ; des céramides ; des agents conservateurs ; des agents opacifiants.

Les adjuvants ci dessus sont en général présents en quantité comprise pour chacun d'eux entre 0,01 et 20% en poids par rapport au poids de la composition.

La composition peut comprendre un ou plusieurs agents épaississants minéraux choisis parmi les argiles organophiles, les silices pyrogénées, ou leurs mélanges.

L'argile organophile peut être choisie parmi la montmorrillonite, la bentonite, l'hectorite, l'attapulgite, la sépiolite, et leurs mélanges. L'argile est de préférence une bentonite ou une hectorite.

Ces argiles peuvent être modifiées avec un composé chimique choisi parmi les amines quaternaires, les amines tertiaires, les acétates aminés, les imidazolines, les savons aminés, les sulfates gras, les alkyl aryl sulfonates, les oxides aminés, et leurs mélanges.

Comme argiles organophiles, on peut citer les quaternium-18 bentonites telles que celles vendues sous les dénominations Bentone 3, Bentone 38, Bentone 38V par la société Rhéox, Tixogel VP par la société United catalyst, Claytone 34, Claytone 40, Claytone XL par la société Southern Clay; les stéaralkonium bentonites telles que celles vendues sous les dénominations Bentone 27 par la société Rheox, Tixogel LG par la société United Catalyst, Claytone AF, Claytone APA par la société Southern Clay ; les quaternium-18/benzalkonium bentonite telles que celles vendues sous les dénominations Claytone HT, Claytone PS par la société Southern Clay ; les quaternium-18/benzalkonium bentonite telles que celles vendues sous les dénominations Claytone HT, Claytone PS par la société Southern Clay, les Quaternium-18 Hectorites telles que celles vendues sous les dénominations Bentone Gel DOA, Bentone Gel ECO5, Bentone Gel EUG, Bentone Gel IPP, Bentone Gel ISD, Bentone Gel SS71, Bentone Gel VS8, Bentone Gel VS38 par la société Rhéox et Simagel M, Simagel SI 345 par la société Biophil.

Les silices pyrogénées peuvent être obtenues par hydrolyse à haute température d'un composé volatil du silicium dans une flamme oxhydrique, produisant une silice finement divisée. Ce procédé permet notamment d'obtenir des silices hydrophiles qui présentent un nombre important de groupements silanol à leur surface. De telles silices hydrophiles sont par exemple commercialisées sous les dénominations "AEROSIL 130®", "AEROSIL 200®", "AEROSIL 255®", "AEROSIL 300®", "AEROSIL 380®" par la société Degussa, "CAB-O-SIL HS-5®", "CAB-O-SIL EH-5®", "CAB-O-SIL LM-130®", "CAB-O-SIL MS-55®", "CAB-O-SIL M-5®" par la société Cabot.

Il est possible de modifier chimiquement la surface de la silice par réaction chimique en vue de diminuer le nombre de groupes silanol. On peut notamment substituer des groupes silanol par des groupements hydrophobes : on obtient alors une silice hydrophobe.

Les groupements hydrophobes peuvent être :
- des groupements triméthylsiloxyl, qui sont notamment obtenus par traitement de silice pyrogénée en présence de l'hexaméthyldisilazane. Des silices ainsi traitées sont dénommées "Silica silylate" selon le CTFA (6ème édition, 1995). Elles sont par exemple commercialisées sous les références "AEROSIL R812®" par la société Degussa, "CAB-O-SIL TS-530®" par la société Cabot.
- des groupements diméthylsilyloxyl ou polydiméthylsiloxane, qui sont notamment obtenus par traitement de silice pyrogénée en présence de polydiméthylsiloxane ou du diméthyldichlorosilane. Des silices ainsi traitées sont dénomées "Silica diméthyl silylate" selon le CTFA (6ème édition, 1995). Elles sont par exemple commercialisées sous les références "AEROSIL R972®", "AEROSIL R974®" par la société Degussa, "CAB-O-SIL TS-610®", "CAB-O-SIL TS-720®" par la société Cabot.

La silice pyrogénée présente de préférence une taille de particules pouvant être nanométrique à micrométrique, par exemple allant d'environ de 5 à 200 nm.

De préférence, la composition comprend une hectorite, une bentonite organomodifiée ou une silice pyrogénée éventuellement modifiée.

Lorsqu'il est présent, l'agent épaississant minéral représente de 1 à 30 % en poids par rapport au poids de la composition.

La composition peut également comprendre un ou plusieurs agents épaississants organiques.

Ces agents épaississants peuvent être choisis parmi les amides d'acides gras (diéthanol- ou monoéthanol-amide de coprah, monoéthanolamide d'acide alkyl éther carboxylique oxyéthyléné), les épaississants polymériques tels que les épaississants cellulosiques (hydroxyéthycellulose, hydroxypropylcellulose, carboxyméthylcellulose), la gomme de guar et ses dérivés (hydroxypropylguar), les gommes d'origine microbienne (gomme de xanthane, gomme de scléroglucane), les homopolymères réticulés d'acide acrylique ou d'acide acrylamidopropanesulfonique et les polymères associatifs (polymères comprenant des zones hydrophiles, et des zones hydrophobes à chaîne grasse (alkyle, alcényle comprenant au moins 10 atomes de carbone) capables, dans un milieu aqueux, de s'associer réversiblement entre eux ou avec d'autres molécules.).

Selon un mode de réalisation particulier, l'épaississant organique est choisi parmi les épaississants cellulosiques (hydroxyéthycellulose, hydroxypropylcellulose, carboxyméthylcellulose), la gomme de guar et ses dérivés (hydroxypropylguar), les gommes d'origine microbienne (gomme de xanthane, gomme de scléroglucane), les homopolymères réticulés d'acide acrylique ou d'acide acrylamidopropanesulfonique, et de préférence parmi les épaississants cellulosiques avec en particulier l'hydroxyéthycellulose.

La teneur en agent(s) épaississant(s) organique(s), s'ils sont présents, varie habituellement de 0,01% à 20% en poids, par rapport au poids de la composition, de préférence de 0,1 à 5% en poids.

Le milieu cosmétiquement acceptable de la composition selon l'invention est un milieu comprenant au moins de l'eau et un ou plusieurs solvants organiques choisis parmi les monoalcools ou les diols, linéaires ou ramifiés, de préférence saturés, comprenant 2 à 10 atomes de carbone, tels que l'alcool éthylique, l'alcool isopropylique, l'hexylèneglycol (2-méthyl 2,4-pentanediol), le néopentylglycol et le 3-méthyl-1,5-pentanediol ; les alcools aromatiques tels que l'alcool benzylique, l'alcool phényléthylique ; le glycérol ; les polyols ou éthers de polyol tels que, par exemple, les éthers monométhylique, monoéthylique et monobutylique d'éthylèneglycol, le 2-butoxyéthanol, le propylèneglycol ou ses éthers tels que, par exemple, le monométhyléther de propylèneglycol, le butylèneglycol, le dipropylèneglycol ; ainsi que les alkyléthers de diéthylèneglycol, notamment en C₁-C₄, comme par exemple, le monoéthyléther ou le monobutyléther du diéthylèneglycol, seuls ou en mélange.

Les solvants représentent généralement entre 1 et 40 % en poids par rapport au poids total de la composition tinctoriale, et de préférence entre 5 et 30 % en poids par rapport au poids total de la composition tinctoriale.

De manière avantageuse, la concentration en eau peut aller de 10 à 70% en poids, de préférence de 20 à 55% en poids, par rapport au poids total de la composition.

La composition tinctoriale selon l'invention peut se présenter sous des formes diverses, telles que sous forme de liquides, de crèmes, de gels, ou sous toute autre forme appropriée pour réaliser une teinture des fibres kératiniques, et notamment des cheveux humains.

Avantageusement, la composition selon l'invention se présente sous la forme d'un gel ou d'une crème.

Le pH de la composition selon l'invention est avantageusement compris entre 3 et 12, de préférence entre 5 et 11. Préférentiellement entre 7 et 11 bornes comprises.

Il peut être ajusté à la valeur désirée au moyen d'agents acidifiants ou alcalinisants habituellement utilisés en teinture des fibres kératiniques ou bien encore à l'aide de systèmes tampons classiques.

Les agents alcalins sont par exemple ceux décrits précédemment.

Parmi les agents acidifiants, on peut citer, à titre d'exemple, des acides minéraux ou organiques comme l'acide chlorhydrique, l'acide ortho-phosphorique, des acides carboxyliques comme l'acide tartrique, l'acide citrique, l'acide lactique, ou des acides sulfoniques.

La composition de l'invention peut être obtenue par mélange d'au moins deux compositions différentes, voire trois ou éventuellement plus de trois compositions différentes. Une ou plusieurs des compositions conduisant par mélange à la composition de l'invention peuvent être anhydres. A noter que la composition selon l'invention est préparée juste avant son application sur les fibres kératiniques humaines.

Par compositions anhydres, on entend plus particulièrement des compositions dont la teneur en eau est égale à 0 ou inférieure à 5% en poids, de préférence inférieure à 2% en poids, et de manière encore plus particulière inférieure à 1% en poids, par rapport au poids de ladite composition. Il est à noter que l'eau peut aussi se trouver sous forme d'eau liée, comme l'eau de cristallisation des sels ou des traces d'eau absorbée par les matières premières utilisées dans la réalisation des compositions selon l'invention.

Selon une variante, la composition selon l'invention est obtenue en mélangeant une première composition comprenant un ou plusieurs corps gras particuliers, un ou plusieurs colorants d'oxydation, avec une deuxième composition comprenant au moins un agent oxydant ; le ou les tensioactifs non ioniques polyoxyéthylénés comprenant de 10 à moins de 80 moles d'oxyde d'éthylène se trouvant dans la première et/ou dans la deuxième compositions et le ou les agents alcalinisants se trouvant également dans la première composition et/ou dans la deuxième composition et de préférence dans la première composition. De préférence ce ou ces tensioactifs non ioniques se trouvent avec la composition comprenant le ou les corps gras.

Selon une deuxième variante de l'invention, la composition selon l'invention est obtenue en mélangeant une première composition comprenant un ou plusieurs corps gras particuliers ; une deuxième composition comprenant un ou plusieurs colorants choisis parmi les colorants d'oxydation ; un ou plusieurs agents alcalinisants ; ou leurs mélanges ; et une troisième composition comprenant au moins un agent oxydant ; le ou les tensioactifs non ioniques polyoxyéthylénés comprenant de 10 à moins de 80 moles d'oxyde d'éthylène se trouvant dans la première, la deuxième et/ou la troisième compositions. La première composition peut en particulier être anhydre. De préférence ce ou ces tensioactifs non ioniques se trouvent avec la composition comprenant le ou les corps gras.

Selon une variante additionnelle de l'invention, la composition selon l'invention est obtenue en mélangeant une première composition comprenant un ou plusieurs corps gras particuliers ; une deuxième composition comprenant un ou plusieurs colorants d'oxydation ; et une troisième composition comprenant au moins un agent oxydant; le ou les tensioactifs non ioniques polyoxyéthylénés comprenant de 10 à moins de 80 moles d'oxyde d'éthylène se trouvant dans la première, la deuxième et/ou la troisième compositions et le ou les agents alcalinisants se trouvant également dans l'une ou l'autre des trois compositions précitées et de préférence dans la première et/ou la seconde composition. La première composition peut en particulier être anhydre. De préférence ce ou ces tensioactifs non ioniques se trouvent avec la composition comprenant le ou les corps gras.

Les ingrédients des compositions précitées et leurs teneurs sont déterminés en fonction des caractéristiques détaillées auparavant pour la composition finale selon l'invention.

Dans chacune des variantes précitées, la composition oxydante est de préférence une composition aqueuse. En particulier, elle comprend plus de 5 % en poids d'eau, de préférence plus de 10% en poids d'eau, et de manière encore plus avantageuse plus de 20% en poids d'eau.

Elle peut également comprendre un ou plusieurs solvants organiques choisis parmi ceux listés auparavant ; ces derniers représentant plus particulièrement, lorsqu'ils sont présents, de 1 à 40% en poids par rapport au poids de la composition oxydante, et de préférence de 5 à 30% en poids.

La composition oxydante comprend également de manière préférée, un ou plusieurs agents acidifiants. Parmi les agents acidifiants, on peut citer à titre d'exemple, les acides minéraux ou organiques comme l'acide chlorhydrique, l'acide orthophosphorique, l'acide sulfurique, les acides carboxyliques comme l'acide acétique, l'acide tartrique, l'acide citrique, l'acide lactique, les acides sulfoniques.

Habituellement, le pH de la composition oxydante, lorsqu'elle est aqueuse, est inférieur à 7.

De préférence, la composition oxydante comprend du peroxyde d'hydrogène en tant qu'agent oxydant, en solution aqueuse, dont le titre varie, plus particulièrement, entre 1 et 40 volumes, et encore plus préférentiellement entre 5 et 40 volumes.

Le procédé de coloration selon l'invention consiste donc à appliquer la composition selon l'invention, sur les fibres kératiniques humaines, sèches ou humides.

La composition est ensuite laissée en place pendant une durée allant habituellement d'une minute à une heure, de préférence de 5 minutes à 30 minutes.

La température durant le procédé est classiquement comprise entre la température ambiante (entre 15 à 25°C) et 80°C, de préférence entre la température ambiante et 60°C.

A l'issue du traitement, les fibres kératiniques humaines sont éventuellement rincées à l'eau, subissent éventuellement un lavage avec un shampooing suivi d'un rinçage à l'eau, avant d'être séchées ou laissées sécher.

Les exemples suivants servent à illustrer l'invention sans toutefois présenter un caractère limitatif.

### EXEMPLES

### EXEMPLE 1

On prépare les compositions suivantes (les quantités sont exprimées en g% de matière active) :

### Composition 1

| | |
|---|---|
| Disteardimonium hectorite (BENTONE 38 VCG) | 3 |
| Octyldodécanol | 13 |
| Huile de vaseline | 77 |
| Carbonate de propylène | 1 |
| Oleth 10 | 6 |

### Composition 2

| | |
|---|---|
| Pentasodium pentetate | 1 |
| Métabisulfite de sodium | 0,7 |
| Monoéthanolamine | 14,5 |
| Toluène-2,5-diamine | 2,25 |
| Chlorhydrate de 2,4-diaminophénoxyéthanol | 0,05 |
| Résorcinol | 2 |
| m-aminophénol | 0,36 |
| Hydroxyéthylcellulose (Natrosol 250 HHR, Aqualon) | 1,5 |
| Hexylène glycol | 3 |
| Dipropylène glycol | 3 |
| Ethanol | 8,25 |
| propylèneglycol | 6,2 |
| Acide ascorbique | 0,25 |
| Eau | Qsp 100 |

### Composition 3

| | |
|---|---|
| Pentasodium pentetate | 0,15 |
| Peroxyde d'hydrogène (solution aqueuse à 50 %) | 12 |
| Sodium stannate | 0,04 |
| Acide phosphorique | Qs pH 2.2 |
| Tétrasodium pyrophosphate | 0,03 |
| Huile de vaseline | 20 |
| Polycondensat tétraméthyl hexaméthylènediamine / dichloro 1,3-propylène (solution aqueuse à 40% Matière Active, Hexadimethrine chloride) | 0,1 |
| Chlorure de poly diméthyl diallyl ammonium (solution aqueuse à 40% non stabilisé, Polyquaternium-6) | 0,2 |
| Glycérine | 0,5 |
| Alcool cétéarylique | 8 |
| Alcool cétylstéarylique oxyéthyléné (33 OE) | 3 |
| Amide d'acides de colza oxyéthyléné (4 OE) | 1,2 |
| Vitamine E : DL-α-Tocophérol | 0,1 |
| Eau | Qsp 100 |

### Mode d'application

Les trois compositions détaillées ci-dessus sont mélangées au moment de l'emploi dans les proportions suivantes :
* 10 g de la composition 1
* 4 g de la composition 2
* 16 g de la composition 3.

Le mélange résultant est ensuite appliqué sur des mèches de cheveux naturels à 90% de blancs, à raison de 10 g de mélange pour 1 g de cheveux.

Le mélange est laissé à température ambiante pendant 30 minutes.

Les cheveux sont ensuite rincés, lavés avec un shampooing standard et séchés.

On obtient des mèches de hauteur de ton châtain clair avec des reflets naturels (évaluation visuelle).

### EXEMPLE 2

On prépare les compositions suivantes (les quantités sont exprimées en g% de matière active) :

### Composition 1

| | |
|---|---|
| Disteardimonium hectorite (BENTONE 38 VCG) | 3 |
| Octyldodécanol | 11,5 |
| Distéarate de glycol | 8 |
| Huile de vaseline | 64,5 |
| Carbonate de propylène | 1 |
| Laureth-2 | 1 |
| Polysorbate 20 | 11 |

### Composition 2

| | |
|---|---|
| Pentasodium pentetate | 1 |
| Métabisulfite de sodium | 0,7 |
| Monoéthanolamine | 14,5 |
| Toluène-2,5-diamine | 2,25 |
| Chlorhydrate de 2,4-diaminophénoxyéthanol | 0,05 |
| Résorcinol | 2 |
| m-aminophénol | 0,36 |
| Hydroxyéthylcellulose (Natrosol 250 HHR, Aqualon) | 1,5 |
| Hexylène glycol | 3 |
| Dipropylène glycol | 3 |
| Ethanol | 8,25 |
| Propylèneglycol | 6,2 |
| Acide ascorbique | 0,25 |
| Eau | Qsp 100 |

### Mode d'application

Les trois compositions détaillées ci-dessus sont mélangées au moment de l'emploi dans les proportions suivantes :
* 10 g de la composition 1
* 4 g de la composition 2
* 16 g de la composition 3.

Le mélange résultant est ensuite appliqué sur des mèches de cheveux naturels à 90% de blancs, à raison de 10 g de mélange pour 1 g de cheveux.

Le mélange est laissé à température ambiante pendant 30 minutes.

Les cheveux sont ensuite rincés, lavés avec un shampooing standard et séchés.

On obtient des mèches de hauteur de ton châtain foncé avec des reflets rouge acajou (évaluation visuelle).

### EXEMPLE 3

### 1- Compositions éclaircissantes (non conformes à l'invention)

On prépare les compositions suivantes (les quantités sont exprimées en g% de matière active) :

### Compositions A1 (invention) et A2 (comparaison)

| Phase | | A1 | A2 |
|---|---|---|---|
| 1 | Myristate d'isopropyle | 87 | 52 |
| 1 | Oleth-10 | 10 | 10 |
| 1 | Eau | 0 | 35 |
| 2 | Disteardiminium hectorite | 2.25 | 2.25 |
| 3 | Propylène carbonate | 0.75 | 0.75 |

### Composition B1

| | |
|---|---|
| Monoéthanolamine | 14,5 |
| Hexylène glycol | 3 |
| Dipropylène glycol | 3 |
| Alcool ethylique | 8,8 |
| Propylène glycol | 6,2 |
| Hydroxyethylcellulose (PM = 1.300.000) | 1,5 |
| Agents réducteurs, agents séquestrants | qs |
| Eau | Qsp 100g |

### Composition C

| | |
|---|---|
| Peroxyde d'hydrogène | 6 |
| Alcool cétéarylique | 2,28 |
| Ceteareth-25 | 0,57 |
| Glycérine | 0,5 |
| Trideceth-2 carboxamide MEA | 0,85 |
| Stabilisants, séquestrants | Qs |
| Acide phosphorique | Qs pH = 2 |
| Eau | Qs 100 |

### Mode d'application

Les trois compositions détaillées ci-dessus sont mélangées au moment de l'emploi dans les proportions suivantes :
- 10g de la composition A1 (invention) ou A2 (comparaison)
- 4g de la composition B1
- 15g de la composition C

Les pH des deux mélanges obtenus sont de 9,9 ± 0,1.

Chaque mélange est ensuite appliqué sur des mèches de cheveux châtains (HT = 4) avec un rapport de bain de 10 g de mélange pour 1 g de cheveux.

Chaque mélange est laissé à 27°C pendant 30 minutes.

A l'issue de ce temps de pause, les mèches sont rincées, lavées avec un shampooing Elsève multivitamines, puis séchées.

Comme le montre le tableau ci-dessous, le mélange de l'invention, comprenant plus de 25% en poids en corps gras conduit à un meilleur éclaircissement que la composition hors invention qui contient moins de 25 % en poids de corps gras.

| | L* | a* | b* | ΔE*ab |
|---|---|---|---|---|
| Cheveu naturel non traité | 21,3 | 3,7 | 4,4 | ---- |
| Cheveu naturel traité avec le mélange de l'invention | 26,4 | 7,4 | 11,4 | 9,4 |
| Cheveu naturel traité avec le mélange hors invention | 23 | 6,4 | 8,8 | 58 |

### 2- Compositions colorantes

On prépare la composition colorante B2 suivante (quantités exprimées en g% de matière active) :

| | |
|---|---|
| Monoéthanolamine | 14,5 |
| Hexylène glycol | 3 |
| Dipropylène glycol | 3 |
| Alcool éthylique dénaturé | 8,8 |
| Propylène glycol | 6,2 |
| Hydroxyéthylcellulose (PM = 1.300.000) | 1,5 |
| Agents réducteurs, agent séquestrant | qs |
| Paraaminophenol | 0.93 |
| Resorcinol | 0.99 |
| 2-amino-3-hydroxypyridine | 0.435 |
| 6-hydroxyindole | 0.26 |
| 2-methylresorcinol | 0.87 |
| 2-methyl-5-hydroxyethylaminophenol | 0.145 |
| Toluène-2,5-diamine | 1.28 |
| Eau | Qsp 100g |

### Mode d'application

Les trois compositions sont mélangées au moment de l'emploi dans les proportions suivantes :
- 10g de la composition A1 (invention) ou A2 (comparative)
- 4g de la composition B2
- 15g de la composition C
Les pH des deux mélanges obtenus sont de 9.8 ± 0.05.

Chaque mélange est ensuite appliqué sur des mèches de cheveux naturels à 90% blancs (BN) et de cheveux permanentés à 90 % blancs (BP), avec un rapport de bain de 10 g de mélange pour 1 g de cheveux.

Chaque mélange est laissé à 27°C pendant 30 minutes.

A l'issue de ce temps de pause, les mèches sont rincées, lavées avec un shampooing Elsève multivitamines, puis séchées.

Comme le montre le tableau ci-dessous, le mélange de l'invention conduit à une coloration plus homogène que la composition hors invention.

| | **L*** | **a*** | **b*** | **Sélectivité BN/BP** |
|---|---|---|---|---|
| BN traité avec le mélange de l'invention | 27,0 | 12,7 | 13,0 | 2,6 |
| BP traité avec le mélange de l'invention | 27,7 | 14,4 | 14,9 | |
| BN traité avec le mélange hors invention | 31,4 | 11,9 | 15,4 | 4,7 |
| BP traité avec le mélange hors invention | 27,5 | 14,0 | 13,9 | |

## Revendications

1. Composition de coloration de fibres kératiniques humaines, **caractérisée en ce qu'**elle comprend, dans un milieu cosmétiquement acceptable:
(a)au moins 25% en poids d'un ou plusieurs corps gras différents des acides gras;
(b)de 1 à 10% en poids d'un ou plusieurs tensioactifs non ioniques comprenant au moins 10 moles et au plus 80 moles d'oxyde d'éthylène ;
(c) un ou plusieurs colorants choisis parmi les colorants d'oxydation;
(d)au moins un agent oxydant ;
(e)un ou plusieurs agents alcalinisants ;
le milieu acceptable comprenant :
(f) de l'eau ; et
(g)au moins un solvant organique choisi parmi les monoalcools ou les diols, linéaires ou ramifiés, de préférence saturés, comprenant 2 à 10 atomes de carbone, tels que l'alcool éthylique, l'alcool isopropylique, l'hexylèneglycol (2-méthyl 2,4-pentanediol), le néopentylglycol et le 3-méthyl-1,5-pentanediol ; les alcools aromatiques tels que l'alcool benzylique, l'alcool phényléthylique ; le glycérol ; les polyols ou éthers de polyol tels que, par exemple, les éthers monométhylique, monoéthylique et monobutylique d'éthylèneglycol, le 2-butoxyéthanol, le propylèneglycol ou ses éthers tels que, par exemple, le monométhyléther de propylèneglycol, le butylèneglycol, le dipropylèneglycol ; ainsi que les alkyléthers de diéthylèneglycol, notamment en C₁-C₄, comme par exemple, le monoéthyléther ou le monobutyléther du diéthylèneglycol, seuls ou en mélange.

2. Composition selon la revendication 1, **caractérisée en ce que** le ou les corps gras sont choisis parmi les composés liquides ou pâteux, et de préférence liquides à température ambiante et à pression atmosphérique.

3. Composition selon l'une quelconque des revendications précédentes dans laquelle le corps gras est choisi parmi les alcanes inférieurs en C₆-C₁₆, les huiles non siliconées d'origine végétale, minérale ou synthétique, les alcools gras, les esters d'acide gras et/ou d'alcool gras, ou leurs mélanges.

4. Composition selon l'une quelconque des revendications précédentes, **caractérisée en ce que** la teneur en corps gras varie de 25 à 80% en poids, de préférence de 25 et 65% en poids, encore plus particulièrement de 30 à 55% en poids, par rapport au poids de la composition.

5. Composition selon l'une quelconque des revendications précédentes, **caractérisée en ce que** le tensioactif non ionique est choisi parmi les composés suivants, seuls ou en mélanges :
• les alkyl(C₈-C₂₄)phénols oxyéthylénés,
• les alcools en C₈-C₃₀, saturés ou non, linéaires ou ramifiés, oxyéthylénés,
• les amides, en C₈-C₃₀, saturés ou non, linéaires ou ramifiés, oxyéthylénés,
• les esters d'acides en C₈-C₃₀, saturés ou non, linéaires ou ramifiés, et de polyéthylèneglycols,
• les esters d'acides en C₈-C₃₀, saturés ou non, linéaires ou ramifiés, et de sorbitol polyoxyéthylénés,
• les huiles végétales oxyéthylénées, saturées ou non ;
le nombre de moles d'oxyde d'éthylène étant d'au moins 10 et allant jusqu'à 80.

6. Composition selon l'une quelconque des revendications précédentes, **caractérisée en ce que** la teneur en tensioactif non ionique est comprise entre 1 et 8% en poids par rapport au poids de la composition.

7. Composition selon l'une quelconque des revendications précédentes, **caractérisée en ce qu'**elle comprend à titre de colorants d'oxydation, une ou plusieurs bases d'oxydation choisies parmi les paraphénylènediamines, les bis-phénylalkylènediamines, les para-aminophénols, les ortho-aminophénols, les bases hétérocycliques et leurs sels d'addition.

8. Composition selon la revendication précédente, **caractérisée** ce qu'elle comprend un ou plusieurs coupleurs choisis parmi les méta-phénylènediamines, les méta-aminophénols, les méta-diphénols, les coupleurs naphtaléniques, les coupleurs hétérocycliques ainsi que leurs sels d'addition.

9. Composition selon l'une quelconque des revendications précédentes, **caractérisée en ce qu'**elle comprend également à titre de colorants directs, des colorants ioniques ou non ioniques, azoïques ; méthiniques ; carbonyles ; aziniques ; nitrés (hétéro)aryle ; tri-(hétéro)aryle méthanes ; les porphyrines ; les phtalocyanines et les colorants directs naturels, seuls ou en mélanges.

10. Composition selon l'une quelconque des revendications précédentes, **caractérisée en ce qu'**elle comprend un ou plusieurs agents alcalinisants choisis parmi l'ammoniaque, les carbonates ou bicarbonates alcalins, les hydroxydes de sodium ou de potassium et les amines organiques dont le pKb à 25°C est inférieur à 12, et de préférence inférieur à 10, encore plus avantageusement inférieur à 6.

11. Composition selon la revendication précédente, **caractérisée en ce que** l'amine organique est une alcanolamine, de préférence la monoéthanolamine.

12. Composition selon la revendication 10, **caractérisée en ce que** l'amine organique est choisie parmi les acides aminés basiques.

13. Procédé de coloration de fibres kératiniques humaines **caractérisé en ce que** l'on applique la composition selon l'une quelconque des revendications précédentes.

## Patentansprüche

1. Zusammensetzung zum Färben oder Aufhellen von menschlichen Keratinfasern, **dadurch gekennzeichnet, dass** sie in einem kosmetisch akzeptablen Medium Folgendes umfasst:
(a) mindestens 25 Gew.-% eines oder mehrerer Fettstoffe, die sich von Fettsäuren unterscheiden;
(b) 1 bis 10 Gew.-% eines oder mehrerer nichtionischer Tenside, umfassend mindestens 10 Mol und höchstens 80 Mol Ethylenoxid;
(c) einen oder mehrere Farbstoffe, ausgewählt aus Oxidationsfarbstoffen;
(d) mindestens ein Oxidationsmittel;
(e) ein oder mehrere Alkalinisierungsmittel;
wobei das akzeptable Medium umfasst:
(f) Wasser und
(g) mindestens ein organisches Lösungsmittel, ausgewählt aus linearen oder verzweigten, vorzugsweise gesättigten Monoalkoholen oder Diolen, umfassend 2 bis 10 Kohlenstoffatome, wie etwa Ethylalkohol, Isopropylalkohol, Hexylenglycol-(2-methyl-2,4-pentandiol), Neopentylglycol und 3-Methyl-1,5-pentandiol; aromatischen Alkoholen, wie etwa Benzylalkohol, Phenylethylalkohol; Glycerin; Polyolen oder Polyolethern, wie zum Beispiel Ethylenglycolmonomethyl-, Ethylenglycolmonoethyl- und Ethylenglycolmonobutylethern, 2-Butoxyethanol, Propylenglycol oder Estern davon, wie zum Beispiel Propylenglycolmonomethylether, Butylenglycol, Dipropylenglycol; sowie den Diethylenglycolalkylethern, insbesondere Diethylenglycol-C₁-C₄-alkylethern, wie zum Beispiel Diethylenglycolmonoethylether oder Diethylenglycolmonobutylether, allein oder als Mischung.

2. Zusammensetzung nach Anspruch 1, **dadurch gekennzeichnet, dass** der oder die Fettstoffe aus flüssigen oder pastösen Verbindungen ausgewählt sind und vorzugsweise bei Umgebungstemperatur und Atmosphärendruck flüssig sind.

3. Zusammensetzung nach einem der vorhergehenden Ansprüche, wobei der Fettstoff ausgewählt ist aus C₆-C₁₆-Niederalkanen, Nicht-Silikonölen pflanzlichen, mineralischen oder synthetischen Ursprungs, Fettalkoholen, Fettsäureestern und/oder Fettalkohol oder Mischungen davon.

4. Zusammensetzung nach einem der vorhergehenden Ansprüche, **dadurch gekennzeichnet, dass** der Fettgehalt zwischen 25 und 80 Gew.-%, vorzugsweise zwischen 25 und 65 Gew.-%, insbesondere zwischen 30 und 55 Gew.-%, bezogen auf das Gewicht der Zusammensetzung, variiert.

5. Zusammensetzung nach einem der vorhergehenden Ansprüche, **dadurch gekennzeichnet, dass** das nichtionische Tensid ausgewählt ist aus den folgenden Verbindungen, allein oder als Mischungen:
• oxyethylenierten Alkyl (C₈-C₂₄) phenolen,
• gesättigten oder ungesättigten, linearen oder verzweigten oxyethylenierten C₈-C₃₀-Alkoholen,
• gesättigten oder ungesättigten, linearen oder verzweigten oxyethylenierten C₈-C₃₀-Amiden,
• gesättigten oder ungesättigten, linearen oder verzweigten C₈-C₃₀-Säureestern und aus Polyethylenglykolen,
• gesättigten oder ungesättigten, linearen oder verzweigten C₈-C₃₀-Säureestern und aus polyoxyethylenierten Sorbitolestern,
• gesättigten oder ungesättigten oxyethylenierten Pflanzenölen;
wobei die Molanzahl an Ethylenoxid mindestens 10 beträgt und bis zu 80 reicht.

6. Zusammensetzung nach einem der vorhergehenden Ansprüche, **dadurch gekennzeichnet, dass** der Gehalt an nichtionischem Tensid zwischen 1 und 8 Gew.-%, bezogen auf das Gewicht der Zusammensetzung, beträgt.

7. Zusammensetzung nach einem der vorhergehenden Ansprüche, **dadurch gekennzeichnet, dass** sie als Oxidationsfarbstoffe eine oder mehrere Oxidationsbasen enthält, die ausgewählt sind aus Paraphenylendiaminen, bis-Phenylalkylendiaminen, para-Aminophenolen, ortho-Aminophenolen, heterocyclischen Basen und Additionssalzen davon.

8. Zusammensetzung nach dem vorhergehenden Anspruch, **dadurch gekennzeichnet, dass** sie ein oder mehrere Kopplungsmittel umfasst, ausgewählt aus meta-Phenylendiaminen, meta-Aminophenolen, meta-Diphenolen, Naphthalin-Kopplungsmitteln, heterocyclischen Kopplungsmitteln und Additionssalzen davon.

9. Zusammensetzung nach einem der vorhergehenden Ansprüche, **dadurch gekennzeichnet, dass** sie als Direktfarbstoffe ionische oder nichtionische Farbstoffe, Azofarbstoffe; Methinfarbstoffe; Carbonylfarbstoffe; Azinfarbstoffe; Nitro-(hetero)arylfarbstoffe; Tri-(hetero)arylmethanfarbstoffe; Porphyrine; Phtalocyanine und natürliche Direktfarbstoffe, allein oder als Mischungen umfasst.

10. Zusammensetzung nach einem der vorhergehenden Ansprüche, **dadurch gekennzeichnet, dass** sie ein oder mehrere Alkalisierungsmittel umfasst, ausgewählt aus wässrigem Ammoniak, Alkalimetallcarbonaten oder -bicarbonaten, Natrium- oder Kaliumhydroxiden und organischen Aminen, deren pKb bei 25 °C kleiner als 12 und vorzugsweise kleiner als 10, noch bevorzugter kleiner als 6 ist.

11. Zusammensetzung nach dem vorhergehenden Anspruch, **dadurch gekennzeichnet, dass** das organische Amin ein Alkanolamin, vorzugsweise Monoethanolamin, ist.

12. Zusammensetzung nach Anspruch 10, **dadurch gekennzeichnet, dass** das organische Amin aus basischen Aminosäuren ausgewählt ist.

13. Verfahren zum Färben von menschlichen Keratinfasern, **dadurch gekennzeichnet, dass** die Zusammensetzung nach einem der vorhergehenden Ansprüche angewendet wird.

## Claims

1. Composition for dyeing human keratin fibres, **characterized in that** it comprises, in a cosmetically acceptable medium:
(a) at least 25% by weight of one or more fatty substances other than fatty acids;
(b) from 1% to 10% by weight of one or more nonionic surfactants comprising at least 10 mol and not more than 80 mol of ethylene oxide;
(c) one or more dyes chosen from oxidation dyes;
(d) at least one oxidizing agent;
(e) one or more basifying agents;
the acceptable medium comprising:
(f) water; and
(g) at least one organic solvent chosen from linear or branched, preferably saturated, monoalcohols or diols, comprising 2 to 10 carbon atoms, such as ethyl alcohol, isopropyl alcohol, hexylene glycol (2-methyl-2,4-pentanediol), neopentyl glycol and 3-methyl-1,5-pentanediol; aromatic alcohols such as benzyl alcohol and phenylethyl alcohol; glycerol; polyols or polyol ethers, for instance ethylene glycol monomethyl, monoethyl or monobutyl ether, 2-butoxyethanol, propylene glycol or ethers thereof, for instance propylene glycol monomethyl ether, butylene glycol, or dipropylene glycol; and also diethylene glycol alkyl ethers, especially C₁-C₄ alkyl ethers, for instance diethylene glycol monoethyl ether or monobutyl ether, alone or as a mixture.

2. Composition according to Claim 1, **characterized in that** the fatty substance(s) are chosen from compounds that are liquid or pasty, and preferably liquid, at room temperature and at atmospheric pressure.

3. Composition according to either of the preceding claims, in which the fatty substance is chosen from C₆-C₁₆ lower alkanes, non-silicone oils of plant, mineral or synthetic origin, fatty alcohols, esters of fatty acid and/or of fatty alcohol, or mixtures thereof.

4. Composition according to any one of the preceding claims, **characterized in that** the content of fatty substance ranges from 25% to 80% by weight, preferably from 25% to 65% by weight and even more particularly from 30% to 55% by weight, relative to the weight of the composition.

5. Composition according to any one of the preceding claims, **characterized in that** the nonionic surfactant is chosen from the following compounds, alone or as mixtures:
• oxyethylenated (C₈-C₂₄) alkylphenols,
• saturated or unsaturated, linear or branched, oxyethylenated C₈-C₃₀ alcohols,
• saturated or unsaturated, linear or branched, oxyethylenated C₈-C₃₀ amides,
• esters of saturated or unsaturated, linear or branched C₈-C₃₀ acids and of polyethylene glycols,
• polyoxyethylenated esters of saturated or unsaturated, linear or branched C₈-C₃₀ acids and of sorbitol,
• saturated or unsaturated, oxyethylenated plant oils;
the number of moles of ethylene oxide being at least 10 and ranging up to 80.

6. Composition according to any one of the preceding claims, **characterized in that** the content of nonionic surfactant is between 1% and 8% by weight relative to the weight of the composition.

7. Composition according to any one of the preceding claims, **characterized in that** it comprises, as oxidation dyes, one or more oxidation bases chosen from para-phenylenediamines, bis(phenyl)alkylenediamines, para-aminophenols, ortho-aminophenols and heterocyclic bases, and the addition salts thereof.

8. Composition according to the preceding claim, **characterized in that** it comprises one or more couplers chosen from meta-phenylenediamines, meta-aminophenols, meta-diphenols, naphthalene-based couplers and heterocyclic couplers, and also the addition salts thereof.

9. Composition according to any one of the preceding claims, **characterized in that** it also comprises, as direct dyes, ionic or nonionic azo; methine; carbonyl; azine; nitro(hetero)aryl; tri(hetero)arylmethane; porphyrin; phthalocyanin dyes and natural direct dyes, alone or as a mixture.

10. Composition according to any one of the preceding claims, **characterized in that** it comprises one or more basifying agents chosen from ammonia, alkali metal carbonates or bicarbonates, sodium hydroxide, potassium hydroxide and organic amines with a pKb at 25°C of less than 12, preferably less than 10 and even more advantageously less than 6.

11. Composition according to the preceding claim, **characterized in that** the organic amine is an alkanolamine, preferably monoethanolamine.

12. Composition according to Claim 10, **characterized in that** the organic amine is chosen from basic amino acids.

13. Process for dyeing human keratin fibres, **characterized in that** the composition according to any one of the preceding claims is applied.
